Europäisches Patentamt

(19) European Patent Office

Office européen des brevets

(11) Numéro de publication: **0 181 252 B1**

(12) **FASCICULE DE BREVET EUROPEEN**

(45) Date de publication du fascicule du brevet:
18.10.89

(21) Numéro de dépôt: **85402027.8**

(22) Date de dépôt: **18.10.85**

(51) Int. Cl.⁴: **C 08 B 37/10, A 61 K 31/725**

(54) Procédé de préparation de compositions de mucopolysaccharides dotées d'une activité antithrombotique élevée.

(30) Priorité: **18.10.84 FR 8415978**
**18.10.84 FR 8415979**

(43) Date de publication de la demande:
**14.05.86 Bulletin 86/20**

(45) Mention de la délivrance du brevet:
**18.10.89 Bulletin 89/42**

(84) Etats contractants désignés:
**AT BE CH DE FR GB IT LI LU NL SE**

(56) Documents cités:
**EP-A- 0 037 319**
**FR-A- 2 440 376**
**FR-A- 2 495 935**
**FR-A- 2 503 714**
**US-A- 4 533 549**

(73) Titulaire: **CHOAY S.A., 48, Avenue Théophile-Gautier, F-75782 Paris Cédex 16 (FR)**

(72) Inventeur: **Lormeau, Jean-Claude, 1, rue Joseph Delattre, F-76150 Maromme (FR)**
Inventeur: **Choay, Jean, 21, rue Saint-Guillaume, F-75007 Paris (FR)**

(74) Mandataire: **Peaucelle, Chantal et al, S.C. Ernest Gutmann - Yves Plasseraud 67, boulevard Haussmann, F-75008 Paris (FR)**

ACTORUM AG

## Description

L'invention a pour objet un procédé de préparation de compositions de mucopolysaccharides dotées d'une activité antithrombotique élevée.

On sait que des compositions de ce type ont été obtenues à partir de l'héparine par extraction alcoolique, ou encore par dépolymérisation par voie chimique ou enzymatique.

D'une manière générale, ces procédés permettent d'obtenir des compositions ou des fractions formées de mucopolysaccharides, ou MPS, dont les chaînes renferment environ 25 à 30 motifs au plus et possèdent une activité anti-Xa, mesurée par le titre Yin Wessler (YW), supérieure à celle de l'héparine et une activité anti-coagulante globale, exprimée par le titre USP, inférieure à celle de l'héparine, ce titre pouvant même présenter une valeur très faible à pratiquement nulle.

Ces produits présentent l'intérêt d'exercer une activité plus spécifique que l'héparine sur certaines étapes mises en jeu dans le processus de coagulation.

Pour un traitement donné, on choisit le produit en fonction de la valeur de son titre YW et du rapport de ses titres YW et USP.

A cet égard, les inventeurs ont recherché des moyens permettant d'obtenir aisément des compositions répondant à un profil donné en ce qui concerne, en particulier, leur titre YW et leur rapport des titres YW à USP. Les travaux effectués les ont conduits à constater qu'en opérant dans des conditions déterminées, il est possible d'isoler de tels produits à partir d'autres compositions de MPS.

L'invention a donc pour but de fournir un nouveau procédé d'obtention de compositions de MPS à activité antithrombotique élevée, basé sur le traitement d'une autre composition de MPS les renfermant, dont la mise en œuvre permet de disposer aisément de produits de grande utilité.

Le procédé de l'invention est caractérisé en ce qu'on soumet des compositions de MPS formées d'une majorité de chaînes de MPS de poids moléculaires (PM) de 1800 à 8000 daltons environ, possédant un titre YW et un rapport des titres YW/USP supérieurs à ceux de l'héparine, à au moins une étape de fractionnement, afin de séparer sélectivement de ces compositions au moins la majeure partie des chaînes de PM inférieur à environ 2000 et celles de PM plus élevé, mais ayant un taux de sulfatation inférieur au taux moyen des chaînes du mélange, et qu'on récupère les fractions débarrassées de ces chaînes. Ces fractions présentent un rapport des titres YW à USP plus faible que celui des compositions de départ mais supérieur à celui de l'héparine avec un titre USP supérieur à celui des compositions de départ.

De préférence, l'étape de fractionnement est effectuée à l'aide d'un mélange a) d'eau renfermant un sel minéral et b) de solvant organique, ce solvant étant choisi parmi ceux dans lesquels au moins la majorité des produits recherchés est sélectivement insoluble et précipité.

Les proportions relatives de sel minéral et de solvant sont ajustées et ce en fonction du pH du milieu pour obtenir la précipitation des chaînes de MPS recherchées.

Selon une disposition préférée de l'invention, le solvant organique est avantageusement un solvant alcoolique, plus spécialement l'éthanol.

Selon une autre disposition préférée, le sel minéral utilisé est constitué, notamment, par un sel miscible dans le solvant organique mis en œuvre, tel que le chlorure de sodium ou de potassium.

Selon une autre disposition préférée, le pH du mélange réactionnel est ajusté à une valeur correspondant à un pH acide, plus spécialement à un pH inférieur à 4.

Selon un mode préféré de réalisation de l'invention, les compositions de MPS mises en œuvre pour le fractionnement comprennent une majorité de chaînes (1) de PM de 1800 à 8000 environ, (2) présentant un rapport des titres YW à USP d'au moins environ 10 et (3) un titre YW d'environ 200 à 250 μmg.

On rappelle que le titre Yin et Wessler est utilisé comme mesure de l'activité anti-Xa selon Yin E. T., Wessler S., BUTLER J. V. dans J. Lab. Clin. Med., 1973, 81, p. 298–310.

Les compositions de MPS utilisées sont avantageusement obtenues selon un procédé de dépolymérisation partielle de l'héparine sous l'action d'un agent chimique tel que l'acide nitreux. On a recours, notamment, au procédé décrit par la demanderesse dans la deuxième demande de certificat d'addition N° 8 006 282 du 20 Mars 1980 au brevet FR-7 831 357 du 6 Novembre 1978.

D'une manière avantageuse, on met en œuvre un procédé de dépolymérisation de ce type, mais basé sur l'autorégulation de la réaction de dépolymérisation, tel que décrit dans la demande de brevet FR-8 107 283 du 10 Avril 1981 au nom de la demanderesse.

Selon l'aspect le plus général de cette dépolymérisation autorégulée, on fait réagir l'héparine et l'acide nitreux, en milieu aqueux, à un pH de l'ordre de 2 à 3, avantageusement de 2,5, en des quantités telles que la concentration d'héparine est d'au moins environ 8% en poids et la molarité de l'acide nitreux de 0,02 à 0,1 M environ, et après arrêt par elle-même de la réaction de dépolymérisation par épuisement de l'acide nitreux, on récupère le mélange de MPS du type de ceux précipitables par un solvant organique.

L'acide nitreux est engendré in situ par addition d'un acide comportant avantageusement des anions biologiquement acceptables, tel que l'acide chlorhydrique ou l'acide acétique, à un dérivé de l'acide nitreux, en particulier un sel, un éther-sel et plus spécialement un sel alcalin ou alcalino-terreux. On utilise plus spécialement du nitrite de sodium selon une molarité de 0,03 à 0,05 M.

Pour la mise en œuvre du procédé selon la présente invention, il est préférable d'utiliser $NaNO_2$ de 0,040 à 0,046 M, plus particulièrement de l'ordre de 0,043 M.

En raison de leur mode d'obtention, les compositions de MPS possèdent à l'extrémité réductrice un motif de structure 2,5-anhydromanno, de préférence choisi parmi les groupes 2,5-anhydromannose, 2,5-anhydromannitol ou acide 2,5-anhydromannonique.

Les compositions de MPS de départ utilisées sont mises en solution à raison de 5% p/v dans de l'eau renfermant 10 g/l de NaCl.

Après ajustement du pH à 3,8, le fractionnement de ces MPS est effectué à l'aide d'un solvant organique permettant de précipiter sélectivement les MPS possédant les plus hauts poids moléculaires et/ou les plus sulfatées du mélange.

Le solvant organique est constitué plus spécialement par un solvant alcoolique, notamment l'éthanol.

L'élimination des chaînes de petit poids moléculaire et de celles de faible taux de sulfate a pour conséquence de conduire à des fractions de MPS de titre USP plus élevé, le titre YW étant pratiquement maintenu.

Les compositions de MPS récupérées lors de la précipitation sont caractérisées d'une manière générale en ce qu'elles sont essentiellement formées de chaînes (1) d'un poids moléculaire moyen de 4000 à 5000 daltons, notamment de 4500 environ, (2) possédant un titre YW d'environ 180 à 200 µ/mg et un rapport du titre YW/USP inférieur à 10, notamment d'environ 6 à 3, plus spécialement de l'ordre de 4.

Ces compositions sont en outre formées d'une majorité de chaînes terminées par des motifs de structure 2,5-anhydromanno lorsque les compositions de départ résultent d'un procédé de dépolymérisation nitreuse de l'héparine.

Les compositions obtenues selon le procédé de l'invention sont dotées d'activités biologiques leur permettant en particulier de contrôler de manière spécifique certaines étapes de la coagulation sanguine.

Elles sont donc particulièrement précieuses pour l'élaboration de médicaments utilisables notamment pour la prévention et le traitement des thromboses et du vieillissement des tissus. Ces médicaments sont utilisés sous les formes d'administration et aux posologies habituelles pour le type d'indications visées.

Les compositions de mucopolysaccharides obtenues sont également utiles pour l'élaboration de réactifs biologiques utilisables au laboratoire, notamment à titre de référence de comparaison pour l'étude d'autres produits dont est testée l'activité anticoagulante, notamment au niveau de l'inhibition du facteur Xa.

En ce qui concerne les produits solubles dans le mélange eau-solvant organique mis en œuvre pour le fractionnement, ils sont dépourvus ou pratiquement dépourvus d'activité antithrombotique.

Ces produits peuvent être récupérés par filtration sur un gel de la fraction soluble. L'utilisation d'une étape de chromatographie d'échanges d'ions permet de séparer de la composition, si on le souhaite, différents types d'oligosaccharides, en particulier des chaînes hexasaccharidiques dotées d'une activité inhibitrice élevée vis-à-vis de la néoangiogénèse.

La récupération des produits à partir des fractions est avantageusement réalisée, par exemple, par précipitation alcoolique et lyophilisation.

D'autres caractéristiques et avantages de l'invention apparaîtront dans les exemples qui suivent. Les exemples 1 et 2 sont relatifs à la préparation de compositions de MPS possédant un rapport des titres YW à USP de l'ordre de 4, les exemples 3 et 4 à l'activité des produits obtenus respectivement selon les exemples 1 et 2, et l'exemple 5 à l'obtention à partir des fractions solubles de produits pratiquement dépourvus d'activité antithrombotique.

Exemple 1

Procédé d'obtention de compositions de MPS possédant un rapport des titres YW/USP de 4 environ.

500 g d'héparine injectable, sous forme de sel de sodium, sont mis en solution dans 4500 ml d'eau déminéralisée, à une température de 18°C.

Le rapport YW/USP de l'héparine utilisée est voisin de 1, ces titres présentant une valeur de l'ordre de 160–170.

La solution obtenue est mise sous agitation énergique, et son pH est abaissé à 2,5 par addition d'acide chlorhydrique concentré. On ajoute alors 15 g de nitrite de sodium dissous dans 300 ml d'eau. Le pH de la réaction est ajusté à 2,5 par de l'acide chlorhydrique concentré, et le volume total de la solution est amené à 5000 ml. On laisse la réaction s'effectuer pendant 45 minutes puis on vérifie l'absence d'ions nitreux résiduels dans la solution réactionnelle, par exemple au moyen de papier indicateur imprégné d'iodure de potassium amidonné (développement d'une coloration bleue-violacée en présence d'ions $NO_2^-$).

On laisse la réaction se poursuivre jusqu'à disparition totale des ions nitreux et absence de réaction au papier iodo-amidonné, en effectuant des contrôles toutes les 3 ou 4 minutes.

Lorsque ces contrôles deviennent négatifs, la réaction est considérée comme étant achevée. Le pH de la solution est alors élevé à 10 à l'aide de soude concentrée, et l'on ajoute 5 g de tétrahydruroborate de sodium.

La solution est maintenue sous agitation pendant 15 heures.

Le tétrahydruroborate de sodium n'ayant pas réagi est détruit par abaissement du pH à 3,0 à l'aide d'acide chlorhydrique concentré. La solution est soumise à agitation 15 minutes, puis le pH est réajusté à 7,0 au moyen de soude concentrée.

Les produits de la réaction sont récupérés par addition de 10 litres d'éthanol. Après 48 heures de repos, le produit est décanté et on élimine le surnageant.

Le précipité est redissous dans 9 litres d'eau déminéralisée. On ajoute 100 g de chlorure de sodium, et on abaisse le pH de la solution à 3,8 à l'aide d'acide chlorhydrique concentré. Le volume est ajusté exactement à 10 litres à l'aide d'eau déminéralisée, et l'on ajoute sous agitation énergique 10 litres d'éthanol. On laisse reposer 48 heures. On siphonne le surnageant que l'on écarte. Le précipité est récupéré, lavé à l'éthanol, broyé, séché sous vide.

On obtient 230 g de produit ayant les caractéristiques suivantes:

Titre USP = 52 ul/mg
Titre Yin et Wessler = 225 ul/mg
Poids moléculaire moyen = 4000 à 5000 daltons.

Exemple 2

Procédé d'obtention de fractions de MPS dotées de titres YW et USP dans un rapport d'environ 4.

5000 g d'héparine injectable sont dissous dans 45 litres d'eau déminéralisée, à 18 °C.

On procède de manière similaire à l'exemple 1 en multipliant les quantités de réactifs par 10, c'est-à-dire:

– en ajoutant 150 g de nitrite de sodium en solution dans 3 litres d'eau,
 – en ajustant le volume de la réaction à 50 litres,
 – en ajoutant 50 g de tétrahydruroborate,
 – en précipitant à l'aide de 100 litres d'éthanol,
 – en redissolvant dans 90 litres d'eau,
 – en ajoutant 1000 g de chlorure de sodium,
 – en ajustant le volume final à 100 litres,
 – en ajoutant finalement 100 litres d'éthanol.

On obtient ainsi 2230 g de produit ayant les caractéristiques suivantes:

Titre USP = 54 ul(mg
Titre Yin et Wessler = 232 ul/mg
Poids moléculaire moyen = 4000 à 5000 daltons.

Dans l'exemple 3 donné ci-après, on rapporte les résultats d'essais pharmacologiques effectués avec le produit de l'exemple 1.

Exemple 3

Etude in vitro de l'activité des produits de l'exemple 1.

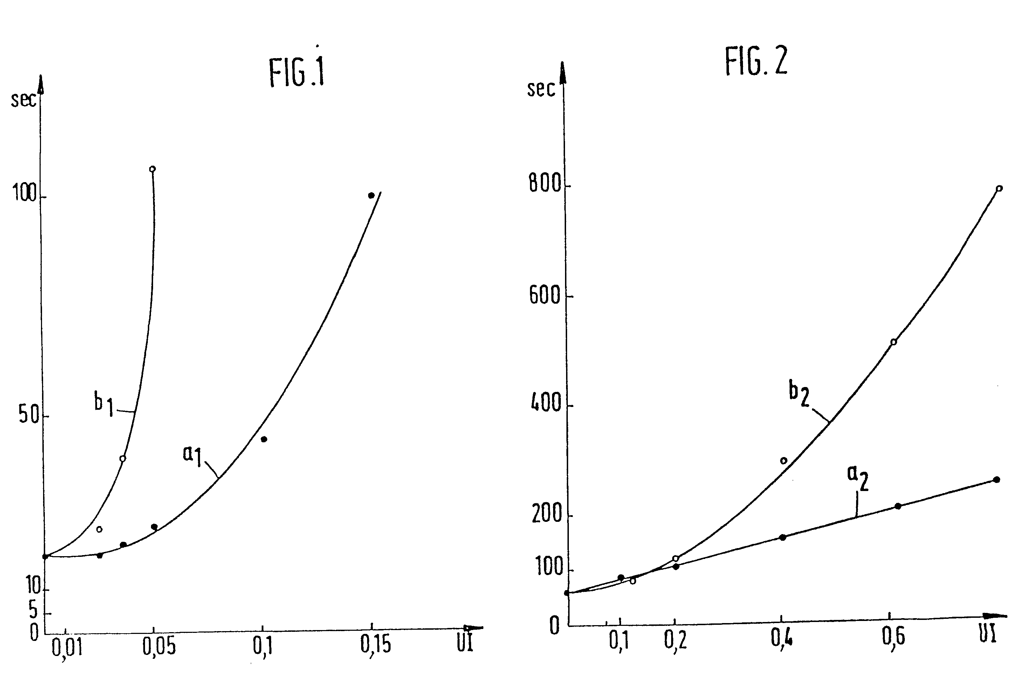

Sur les figures 1 à 4, on rapporte les courbes obtenues en étudiant, in vitro, la variation des temps de coagulation induits dans des plasmas sanguins humains par des doses croissantes d'une héparine du commerce et du produit de l'exemple 1.

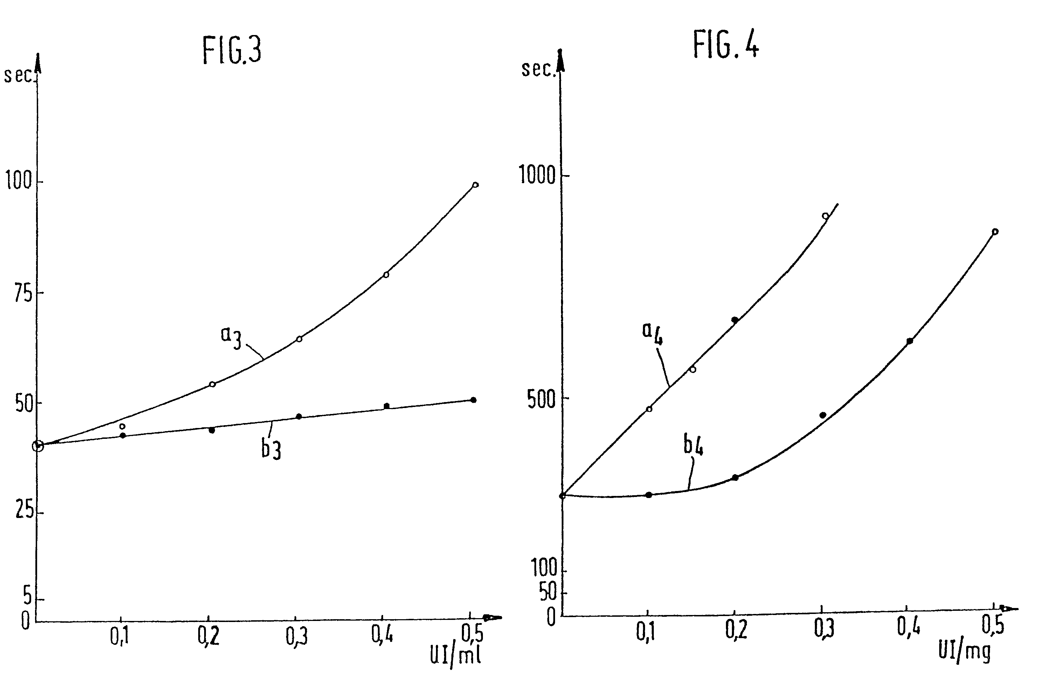

Dans les essais correspondant aux résultats donnés dans les figures 3 et 4, on a utilisé des plasmas exempts de plaquettes, et appauvris en facteur XIA.

Ces figures font apparaître les variations en secondes des temps de thrombine (figure 1), de céphaline-kaolin (figure 2), de coagulation en présence de thromboplastine concentrée (figure 3) et de thromboplastine diluée (figure 4), induits par les produits testés, à savoir le produit de l'exemple 1 (courbes a1, a2, a3 et a4) et l'héparine (courbes b1, b2, b3 et b4) en fonction des doses respectivement utilisées.

Les temps de thrombine et les temps de céphaline-kaolin constituent tous deux des types de mesure reflétant plutôt l'action des préparations étudiées respectivement sur l'inhibition du facteur II activé et la coagulation globale. L'étude des courbes des figures 1 et 2 montre que les MPS selon l'invention exercent un effet nettement plus réduit que celui de l'héparine sur l'inhibition de l'activation de la prothrombine et au niveau de la coagulation globale. Les figures 3 et 4 qui sont représentatives de phénomènes plus directement liés à la séquence des réactions enzymatiques, caractéristiques de la coagulation extrinsèque (notamment en l'absence relative du facteur XIa), font apparaître l'effet avantageux des MPS de l'invention par rapport à l'héparine.

L'exemple 4 ci-dessous concerne les résultats d'essais in vivo chez le lapin avec les MPS de l'exemple 2.

Exemple 4

Etude de l'activité antithrombotique du produit de l'exemple 2 chez le lapin.

L'administration de 500 µ YW au lapin provoque une activité anti-Xa importante alors que l'activité anticoagulante globale reste relativement faible.

Deux heures après l'administration, l'activité YW est de l'ordre de 0,88 µ/ml alors que le temps de céphaline-kaolin est de 0,09 µl/ml.

Les essais in vitro et in vivo montrent donc l'action nettement plus sélective des MPS de l'invention notamment au niveau de l'inhibition du facteur Xa, que celle de l'héparine.

Exemple 5

Procédé d'obtention de fractions de MPS pratiquement dépourvues d'activité antithrombotique.

On récupère la fraction soluble de l'exemple 1. Cette fraction renferme des chaînes de 2 à 14 motifs.

Une étape de fractionnement alcoolique supplémentaire de cette fraction conduit à l'obtention d'un précipité, qui est séparé, et à une fraction soluble à partir de laquelle on récupère la composition y. Cette composition est essentiellement formée de chaînes de 6 à 8 motifs au plus et présente un titre YW inférieur à 70 et un titre USP inférieur à 5.

En soumettant la composition y à une étape de filtration sur gel selon les techniques classiques, on récupère des compositions hexasaccharidiques. Ces compositions sont pratiquement totalement dépourvues d'activité anticoagulante globale et d'activité YW.

Exemple 6

Activité sur la néoangiogénèse des compositions y et z.

Les essais in vitro relatifs à l'activité antiangiogénèse ont été réalisés sur la membrane chorioallantoïque d'embryons de poulets de 6 jours en culture dans des récipients de pétri.

On forme des pastilles en mélangeant 5 µg d'hydrocortisone à 10 µl de méthylcellulose, puis on ajoute les compositions y et z, selon des doses allant de 5 µg à 100 µg. Les pastilles formées sont séchées puis placées sur la membrane chorioallantoïque à laquelle elles adhèrent. Pour chaque essai, on utilise 4 œufs.

Après 48 heures d'incubation, on examine la présence des zones avasculaires. On observe une diminution importante des zones vasculaires. Ces résultats sont obtenus d'une manière remarquable avec seulement 6 µg des produits testés.

Exemple 7

Action de la composition hexasaccharidique z sur un mélanome de type B 16 de souris.

On effectue les essais sur des souris C57 BL/6 porteuses de mélanomes B 16.

Le traitement est effectué sur 15 jours. On administre à chaque souris par injection sous-cutanée deux fois par jour, 0,15 mg de compositions d'hexasaccharides et de l'acétate de cortisone selon des doses allant en diminuant durant le traitement de 250 mg/kg/j à 40–50 mg/kg/j. A la fin des 15 jours, on constate un arrêt du développement des tumeurs.

Une régression de ces tumeurs est même avantageusement observée.

**Revendications**

1. Procédé de préparation de mucopolysaccharides ou MPS possédant une activité antithrombotique élevée, caractérisé en ce qu'on soumet des compositions formées d'une majorité de chaînes de MPS de PM de 1800 à 8000 daltons environ, possédant un titre YW et un rapport des titres YW/USP supérieurs à ceux de l'héparine, à au moins une étape de fractionnement, afin de séparer sélectivement de ces compositions au moins la majeure partie des chaînes de PM inférieur à environ 2000 et celles de PM plus élevé, mais ayant un taux de sulfatation inférieur au taux moyens des chaînes du mélange, et qu'on récupère les fractions débarassées de ces chaînes, ces fractions présentant un rapport des titres YW à USP plus faible que celui des compositions de départ mais supérieur à celui de l'héparine avec un titre USP supérieur à celui des compositions de départ.

2. Procédé selon la revendication 1, caractérisé en ce que le fractionnement est effectué à l'aide d'un mélange d'eau renfermant un sel minéral et de solvant organique dans lequel au moins la majorité des produits recherchés est sélectivement insoluble.

3. Procédé selon la revendication 2, caractérisé en ce qu'on effectue le fractionnement à l'aide d'un solvant organique permettant de précipiter sélectivement les MPS formés de chaînes de PM moyen de 4000 à 5000 daltons, notamment de l'ordre de 4500, possédant un rapport des titres YW à USP inférieur à environ 10, avec un titre YW d'environ 180 à 200 u/mg.

4. Procédé selon la revendication 3, caractérisé en ce que le rapport des titres YW à USP des MPS précipités est de 6 à 3 environ, notamment voisin de 4.

5. Procédé selon l'une quelconque des revendications 2 à 4, caractérisé en ce que le solvant organique est un solvant alcoolique.

6. Procédé selon la revendication 5, caractérisé en ce que le solvant alcoolique est l'éthanol.

7. Procédé selon l'une quelconque des revendications 2 à 6, caractérisé en ce que le sel minéral utilisé est constitué par un sel miscible dans le solvant organique mis en œuvre, tel que le chlorure de sodium ou de potassium.

8. Procédé selon l'une quelconque des revendications 2 à 7, caractérisé en ce qu'on ajuste le pH du mélange réactionnel à une valeur correspondant à un pH acide.

9. Procédé selon la revendication 8, caractérisé en ce que le pH est inférieur à 4.

10. Procédé selon la revendication 1, caractérisé en ce qu'on met en solution dans de l'eau 5% p/v de compositions de MPS renfermant 10 g/l de NaCl et qu'après ajustement du pH à 3,8, on effectue le fractionnement par addition d'un volume d'éthanol et qu'on récupère le précipité formé.

11. Procédé selon l'une quelconque des revendications 1 à 10, caractérisé en ce que les compositions de MPS mises en œuvre pour le fractionnement possèdent un rapport des titres YW à USP d'au moins environ 10 et un titre YW d'environ 200 à 250 u/mg.

12. Procédé selon l'une quelconque des revendications précédentes, caractérisé en ce que les compositions de MPS de départ possèdent à l'extrémité réductrice un motif de structure 2,5-anhydromanno choisi de préférence parmi les groupes 2,5-anhydromannose, 2,5-anhydromannitol ou acide 2,5-anhydromannonique.

**Claims**

1. Process for preparing mucopolysaccharides or MPS possessing high antithrombotic activity, characterized by the fact that compositions formed form a majority of MPS chains having a molecular weight of about 1800 to 8000 daltons, a YW titer and a ratio of the YW/USP titers higher than those of heparin to at least one fractionation step, in order to selectively separate from said compositions at least the major part of chains having MW lower than about 2000 and those having a higher MW but a sulfate content lower than the sulfate average contents of the mixture chain and that the fractions devoid from said chains are recovered, said fractions having a lower ratio of YW/USP titers than the starting compositions but higher than the one of heparin with USP titer higher than the one of the starting compositions.

2. A process according to claim 1, wherein the fractionation is carried out by means of a mixture of water containing an inorganic salt and of an organic solvent in which at least the majority of the desired products is selectively insoluble.

3. A process according to claim 2, wherein the fractionation is carried out by means of an organic solvent enabling the selective precipitation of MPS formed of chains having an average MW of 4000 to 5000 daltons, particularly of about 4500, having a ratio of the YW to USP titers less than about 10, with a YW titer of about 180 to 200 u/mg.

4. A process according to claim 3, wherein the ratio of the YW to USP titers of the precipitated MPS is of about 6 to 3, particularly in the vicinity of 4.

5. A process according to anyone of claims 2 to 4, wherein the organic solvent is an alcoholic solvent.

6. A process according to claim 5, wherein the alcoholic solvent is ethanol.

7. A process according to anyone of claims 2 to 6. wherein the inorganic salt is constituted by a salt miscible in the organic solvent used, such as sodium or potassium chloride.

8. A process according to anyone of claims 2 to 7, wherein the pH of the reaction mixture is adjusted to an acid value.

9. A process according to claim 8, wherein the pH is below 4.

10. A process according to claim 1, comprising placing in solution in water 5% w/v of MPS containing 10 g/l of NaCl and after adjustment of the pH to 3.8, effecting the fractionation by the addition of one volume of ethanol and recovering the precipitate formed.

11. A process according to anyone of claims 1 to 10, wherein the MPS compositions employed for the fractionation possess a ratio of the YW to USP titers of at least about 10 and a YW titer of about 200 to 250 u/mg.

12. A process according to anyone of the preceding claims, wherein the MPS compositions employed possess at the reducing end a unit of 2,5-anhydromanno structure selected preferably from among the groups 2,5-anhydromannose, 2,5-anhydromannitol or 2,5-anhydromannonic acid.

## Patentansprüche

1. Verfahren zur Herstellung von Mucopolysacchariden oder MPS mit erhöhter antithrombotischer Aktivität, dadurch gekennzeichnet, daß man Verbindungen, die aus einem Hauptanteil an MPS-Ketten mit einem Molekulargewicht von ca. 1800 bis 8000 Dalton gebildet sind und einen YW-Titer und ein Verhältnis von YW-Titer zu USP-Titer über denen des Heparins aufweisen, wenigstens einer Fraktionierungsstufe unterwirft, um von diesen Verbindungen wenigstens den Hauptanteil der Ketten mit einem Molekulargewicht von unter ca. 2000 und solche mit einem höheren Molekulargewicht selektiv abzutrennen, die jedoch einen Sulfatisierungsgrad aufweisen, der unter dem durchschnittlichen Sulfatisierungsgrad der Ketten der Verbindung liegt, und daß man die von diesen Ketten befreiten Fraktionen abtrennt, wobei diese Fraktionen ein Verhältnis von YW-Titer zu USP-Titer aufweisen, das unter dem der Ausgangsgemische liegt, jedoch über dem des Heparins bei einem USP-Titer über dem der Ausgangsgemische.

2. Verfahren nach Anspruch 1, dadurch gekennzeichnet, daß die Fraktionierung mit Hilfe einer Mischung aus ein Mineralsalz enthaltendem Wasser und einem organischen Lösungsmittel, in dem wenigstens der Hauptanteil der erwünschten Produkte selektiv löslich ist, durchgeführt wird.

3. Verfahren nach Anspruch 2, dadurch gekennzeichnet, daß man die Fraktionierung mit Hilfe eines organischen Lösungsmittels durchführt, das die selektive Ausfällung der aus Ketten mit einem durchschnittlichen Molekulargewicht von 4000 bis 5000 Dalton bestehenden MPS, insbesondere in der Größenordnung von 4500, mit einem Verhältnis des YW-Titers zum USP-Titer von unter ca. 10, bei einem YW-Titer von ca. 180 bis 200 E/mg ermöglicht.

4. Verfahren nach Anspruch 3, dadurch gekennzeichnet, daß das Verhältnis des YW-Titers zum USP-Titer der ausgefällten MPS ca. 6 bis 3 beträgt, insbesondere annähernd 4.

5. Verfahren nach einem der Ansprüche 2 bis 4, dadurch gekennzeichnet, daß das organische Lösungsmittel ein alkoholisches Lösungsmittel ist.

6. Verfahren nach Anspruch 5, dadurch gekennzeichnet, daß das alkoholische Lösungsmittel Ethanol ist.

7. Verfahren nach einem der Ansprüche 2 bis 6, dadurch gekennzeichnet, daß das verwendete Mineralsalz ein mit dem verwendeten organischen Lösungsmittel mischbares Salz wie Natrium- oder Kaliumchlorid ist.

8. Verfahren nach einem der Ansprüche 2 bis 7, dadurch gekennzeichnet, daß man den pH des Reaktionsgemisches auf einen Wert einstellt, der einem sauren pH entspricht.

9. Verfahren nach Anspruch 8, dadurch gekennzeichnet, daß der pH unter 4 liegt.

10. Verfahren nach Anspruch 1, dadurch gekennzeichnet, daß man 5 Vol.-% MPS-Gemische in 10 g/l NaCl enthaltendem Wasser löst und nach Einstellung des pH's auf 3,8 durch Zugabe eines bestimmten Volumens Ethanol fraktioniert und den erhaltenen Niederschlag abtrennt.

11. Verfahren nach einem der Ansprüche 1 bis 10, dadurch gekennzeichnet, daß die für die Fraktionierung verwendeten MPS-Verbindungen ein Verhältnis des YW-Titers zum USP-Titer von wenigstens ca. 10 bei einem YW-Titer von ca. 200 bis 250 E/mg aufweisen.

12. Verfahren nach einem der vorhergehenden Ansprüche, dadurch gekennzeichnet, daß die Ausgangs-MPS-Verbindungen an ihrem reduzierenden Ende eine 2,5-Anhydromannogruppe, vorzugsweise ausgewählt unter den Gruppen 2,5-Anhydromannose, 2,5-Anhydromannitol oder 2,5-Anhydromannonsäure, aufweisen.

FIG.1

FIG.2

EP 0 181 252 B1

FIG.3

FIG.4

EP 0 181 252 B1